# EUROPEAN PATENT APPLICATION

(11) **EP 1 211 467 A1**
(43) Date of publication of application: **05.06.2002**
(21) Application number: 99956373.7
(22) Date of filing: 13.08.1999
(51) Int. Cl.: F24J 1/00

(54) **INDIVIDUAL AUTONOMOUS HEATER**

(71) Applicant: THE PROCTER & GAMBLE COMPANY, Cincinnati, Ohio 45202 (US)
(72) Inventor: DYATLOV, Valery Alexandrovich, Moscow, 119034 (RU); HARTMUT, Hoehne, D-65842 Schwalbach am Taunus (DE); VINOGRADOV, Valentin Antonovich, Moscow, 123007 (RU); KRENEV, Vladimir Alexandrovich, Moscow, 109377 (RU); DROBOT, Nataliya Fedorovna, Moscow, 125319 (RU); GAVRICHEV, Konstantin Sergeevich, Moscow, 117334 (RU); BABIEVSKAYA, Irina Zinovievna, Moscow, 117454 (RU); NOSKOVA, Olga Anatolievna, Moskovskaya obl., 144007 (RU); BAZHENOVA, Elena Vladimirovna, Moscow, 127238 (RU); VASILIEV, Viktor Sergeevich, Moscow, 125422 (RU)
(74) Representative: Cabinet Hirsch
(86) International application number: RU9900293
(87) International publication number: WO0113048

(57) **Abstract**

The present invention relates to a new individual autonomous heating device comprising, in particular, an exothermic material as a source of heat and a flexible heat-distributing layer positioned between the source of heat and the object being heated, wherein the heat-distributing layer is made of a material with anisotropic heat conductivity creating a primarily lateral heat flow, with a relative lateral heat conductivity within the range of 1.6-0.4 J/(°C^{.}cm^{.}sec).

## Description

### Field of the Invention

The invention relates to an autonomous heating device in the form of an individual package comprising, in particular, exothermic material as a source, which is placed between two layers: an outer, contacting with air and the source of heat, and a flexible heat distributing layer that is placed between the source of heat and the object being heated. This heating device may be used, for example, as a medical heating pad, to heat food products and beverages, etc.

### Background of the Invention

In recent years a great deal of attention has been devoted to developing and using heat generating materials and articles based thereon. For example, phase change materials, capable of storing and generating heat, have found wide use in building materials, materials for road surfaces, containers for beverages and food products, in medical heating pads and in textile articles, for example, in clothes.

For example, seat pads were proposed [USA patent 4995126, 1991, IPC5 A47C 21/04]. Powders of an oxidized metal and a water-containing material, which are arranged in the form of an inner layer between two outer shells, one of which ensuring the access of air, are used in those pads as a disposable exothermic material, i.e. a material capable of generating heat.

An appliance for heating food is also known in which there is an exothermic material comprising powder of an easily corroded Mg-Fe alloy, which is activated by water, and other additives [USA patent 5117809, 1992, IPC⁵ F24J 1/00]. Of importance during the development of this appliance are the requirements stipulated in respect of the shell in which the heat-generating material is disposed.

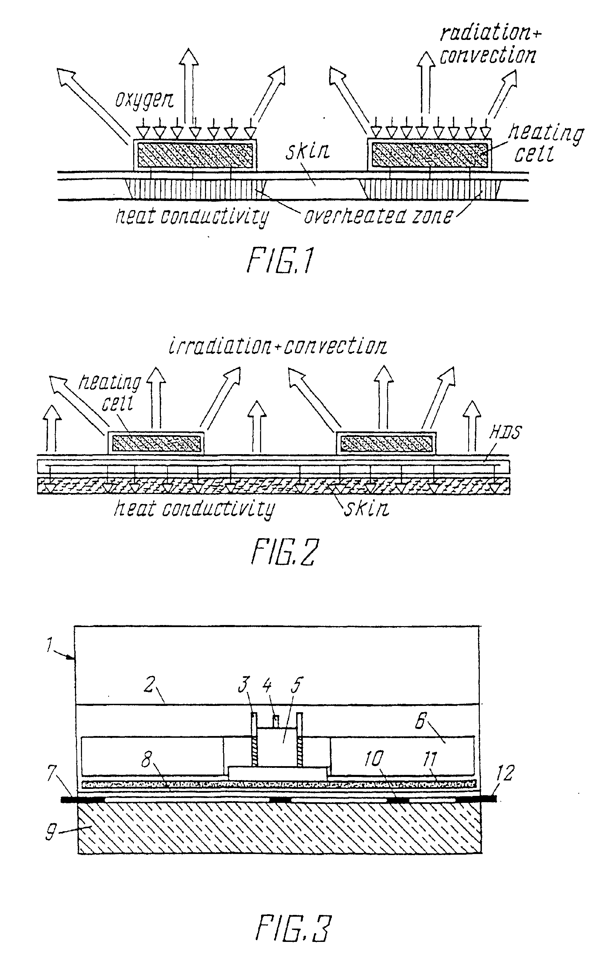

Quite often, problems of heating devices are the nonuniform heating over the plane of the heating device and the possibility of local overheating that is due to the high heat-generating capability of the source of heat and the low heat conductivity of the object being heated, in particular, a human body. For example, when a medical heating pad is used in which the source of heat is concentrated in a small volume, high heating, even a burn, may occur on one portion of the part of the body being heated, while other portions that require heating remain unheated. A diagram of the heat flows in pads of known constructions is shown in Fig. 1.

One more problem may be the requirement that the heating device should be sufficiently flexible so that maximum contact with the object being heated is achieved. In the case of medical heating pads, for example, it is desirable that it have maximum contact with the human body.

These problems may be solved by different methods. For example, one method could be distribution of the source of heat over a wider surface of the heating device. However, such a method cannot always be used, for example, in the case where it is not possible to distribute or place the source of heat over a large surface or where such placement results in a substantial loss in the flexibility of a heating device for which that flexibility is obligatory.

Therefore, the development of a heating device, which has the capability of uniformly generating heat over the whole heating surface and which is also sufficiently flexible, is a timely problem.

Thus, the object of the present invention is to develop such a heating device, the heating surface of which would have good lateral heat conductivity and would be sufficiently flexible.

### Disclosure of the Invention

Thus, the present invention relates to a new, individual, autonomous heating device comprising, in particular, exothermic material as the source of heat and a flexible heat-distributing layer that is positioned between the source of heat and object being heated.

There are no special requirements in respect of the outer insulating layer, and therefore any material, which is suitable for use with a concrete source of heat, may be used as the material for that layer. For example, in the case of a heat-generating source on the base of a phase-change material, the material of the outer insulating layer should be airtight, while in the case of oxidized powders of metals or alloys, the material should ensure adjustable access of air to the source of heat.

A heat-generating composition, for example, on the base of a phase-change material, may be used as the exothermic material [USSR patent 1833404 A3, priority date - 6 February 1990, IPC⁵ C 09 K /06].

The authors, as a result of intensive research, have established that the desired positive effect may be achieved when a material with anisotropic heat conductivity that creates a primarily lateral heat flow is used. The diagram of heat flows in the case of use of heat-distributing layers is shown in Fig. 2.

It is preferable that the heat conductivity of the aforesaid material be within the range of from 1.6 to 0.4 J/(°C^{.}cm^{.}sec).

Materials that are suitable for the objects indicated above may be, for example, metals, woven and nonwoven materials and combinations thereof.

Aluminum foil having a thickness of 0.04 mm, for example, may be indicated as a metal for the flexible heat-distributing layer.

In order to provide gas- and moisture-exchange of the heat-distributing layer, the aforesaid foil may be perforated.

A brass grid of 0.2 mm wire may also be used.

The material for the heat-distributing layer may also be woven polymer material, with finely dispersed carbon particles included in the composition thereof.

An example of woven material is also such a woven material as Viskun, woven carbon textile, woven material RVTU.

Finely dispersed particles of metals may be included in the woven material for the heat-distributing layer.

Such a metal is, for example, nickel. The following are examples of such a material:
- Woven polyester material, in which there are finely dispersed particles of nickel with a specific resistance of 1.5 ohms included in oriented fibers;
- Woven material arimide, in which there are finely dispersed particles of nickel with a specific resistance of 0,8 ohm included in oriented fibers;
- Woven material Phenylon, in which there are finely dispersed particles of nickel with a specific resistance of 0.7 ohm included in oriented fibers.

An example of a material based on aluminum is, for example, woven polymer material, in which there are finely dispersed particles of aluminum (50% by weight) included in oriented fibers.

The material for the heat-distributing layer may be an nonwoven polymer material with randomly oriented fibers in which finely dispersed (5-20 µm) particles of carbon (50% by weight) are included for better heat conductivity.

Such carbon may be, for example, BAU carbon, AG-3 carbon, SKT-125 carbon, SKT-250 carbon.

Examples of nonwoven materials comprising carbon are, for example, nonwoven polymer material in which finely dispersed (5-20 µm) particles of BAU carbon are included; nonwoven polymer material in which finely dispersed (5-20 µm) particles of AG-3 carbon are included; nonwoven polymer material in which finely dispersed (5-20 µm) particles of SKT-125 carbon (surface density 125 g/m²) are included; nonwoven polymer material in which finely dispersed (5-20 µm) particles of SKT-250 carbon (surface density 250 g/m²) are included.

The heat-distributing layer may also be modified by dividing it into two layers: a layer that conducts heat well and is in direct contact with the source of heat and a layer that moderately conducts heat and is in contact with the object being heated. In this manner it becomes possible to distribute heat more uniformly over a large surface.

Aluminum foil having a thickness of 0.04 mm may be used as the layer that conducts heat well, and woven carbon fabric material with a relative lateral heat conductivity of about 0.7/0.7 as the layer that moderately conducts heat.

Moreover, in order to obtain maximum efficient use of heat generated by a source of heat on the base of phase-change material, it may be proposed that a heat-insulating layer be used as the outer insulating layer.

Use of such an outer heat-insulating layer makes it possible to minimize the radiation and convection components of heat exchange, this ensuring more uniform heat distribution.

Nonwoven material made of polyacrylonitrile may be used as the heat-insulating layer, and material for the heat-conducting layer may be, for example, aluminum foil (0.015 mm); carbon textile material TU; Viskun; RVTU carbon textile material; woven polyester material, in the oriented fibers of which finely dispersed particles of nickel are included; nonwoven with filling of finely dispersed boron nitride.

Use of the proposed heating device as, for example, a medical heating pad makes it possible to completely avoid the possibility of a burn when a phase-change material is used as the source of heat. Wherein, the heating pad maintains the flexibility necessary for maximum contact with a human body, and also does not practically differ in weight from a heating pad without the proposed layer.

The examples presented below illustrate the proposed invention, but do not in any way limit the invention.

### Experimental Part

The proposed materials were studied on an installation with a measuring cell shown in Fig. 3. The conditions of heat exchange in this construction to a maximum degree correspond to an actual autonomous, individual medical heating pad device. An electrical heater is used as the heating cell (HC). The power of the heater corresponds to the power of a source of heat based on phase-change material. During the first stage, several thermocouples were placed under the heater to determine its temperature profile. A heater produced at the present time by the P&G firm, in which the source of heat generation was replaced by a heating cell, was used as a model of a medical heating pad.

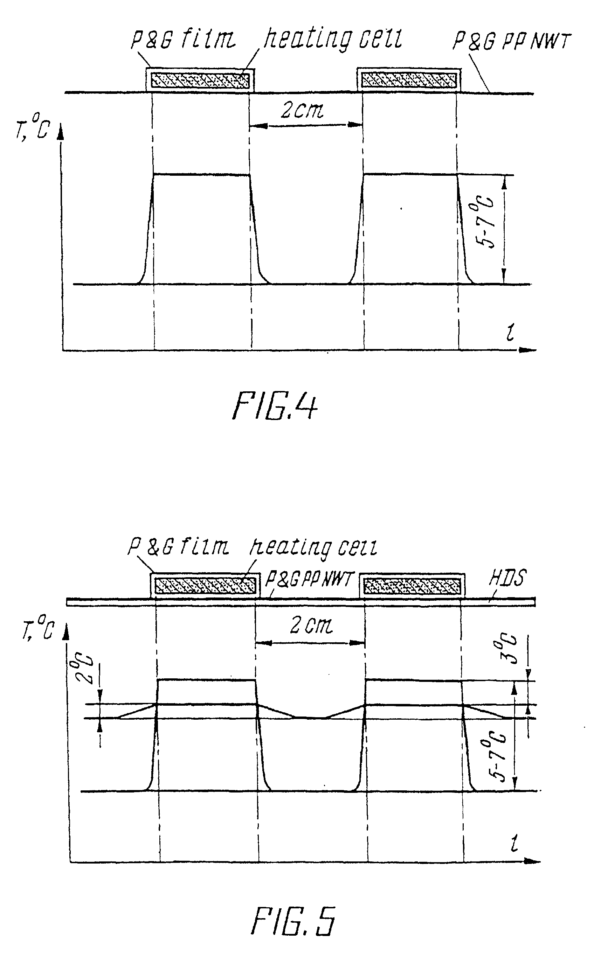

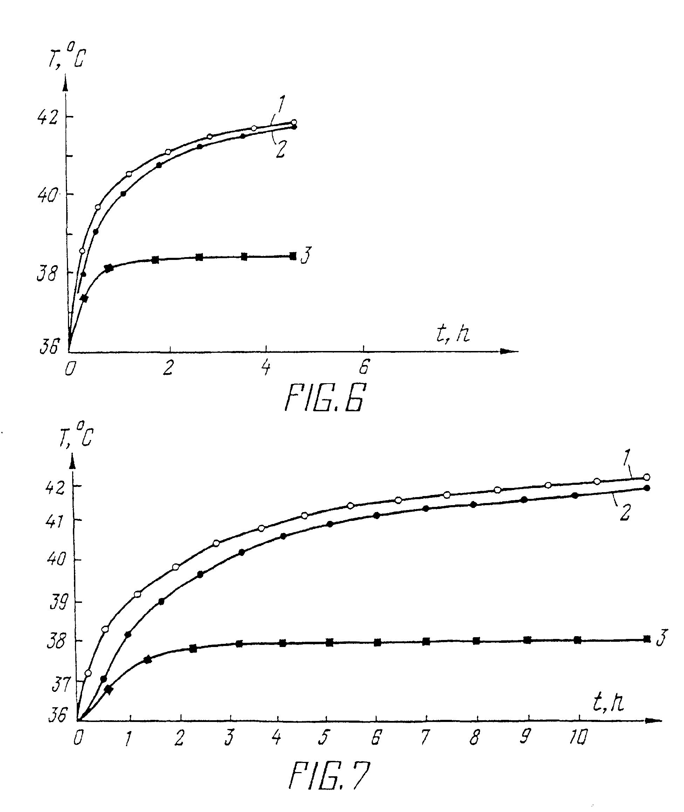

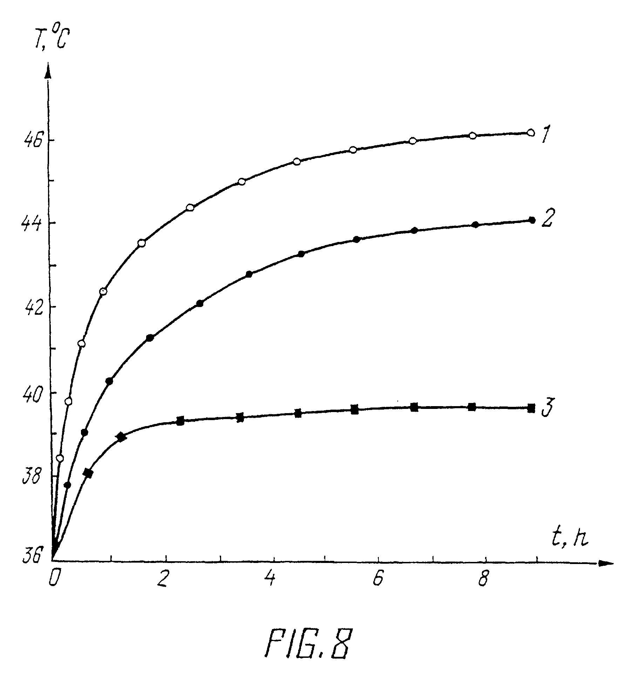

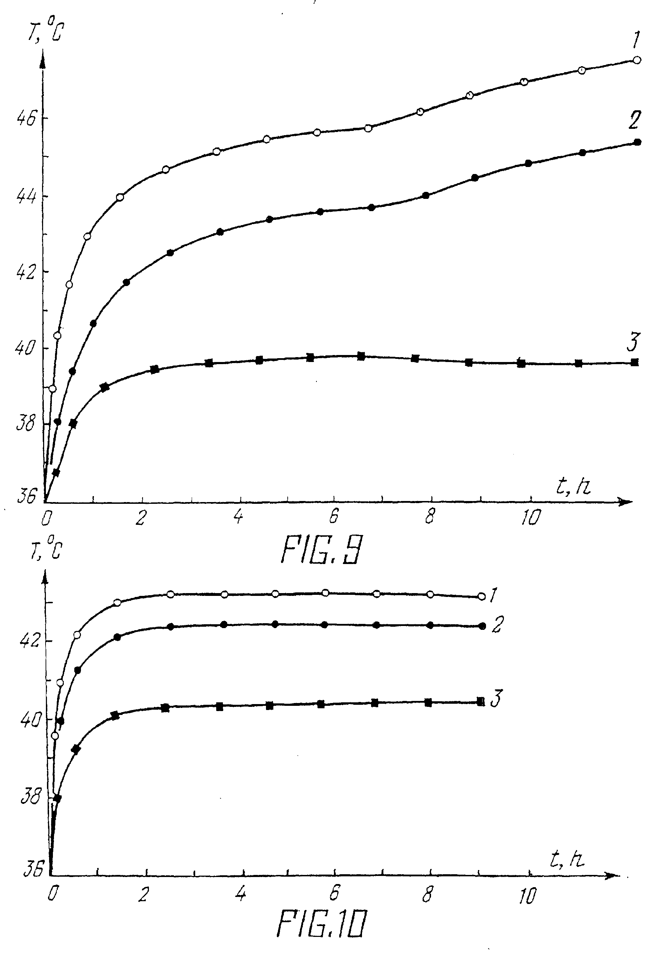

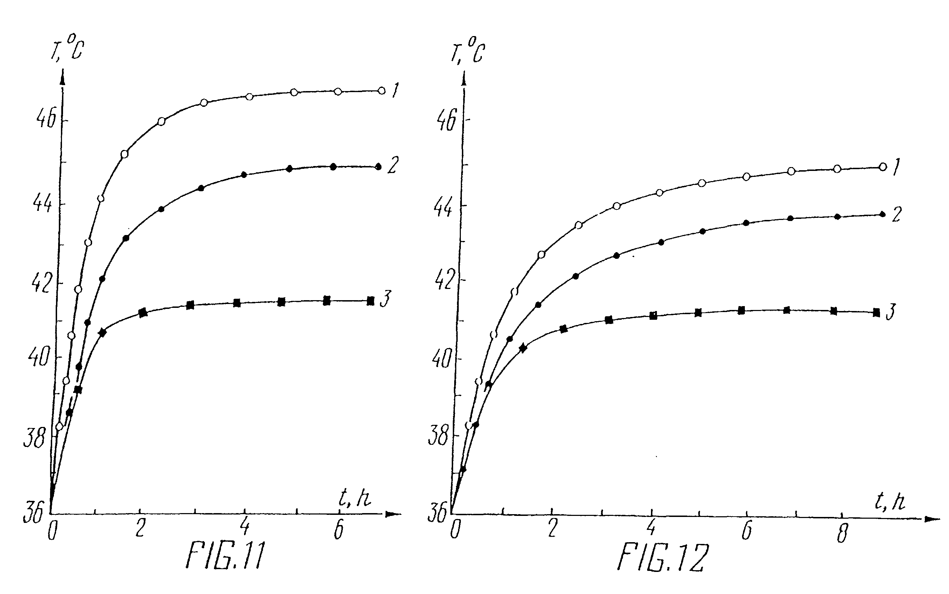

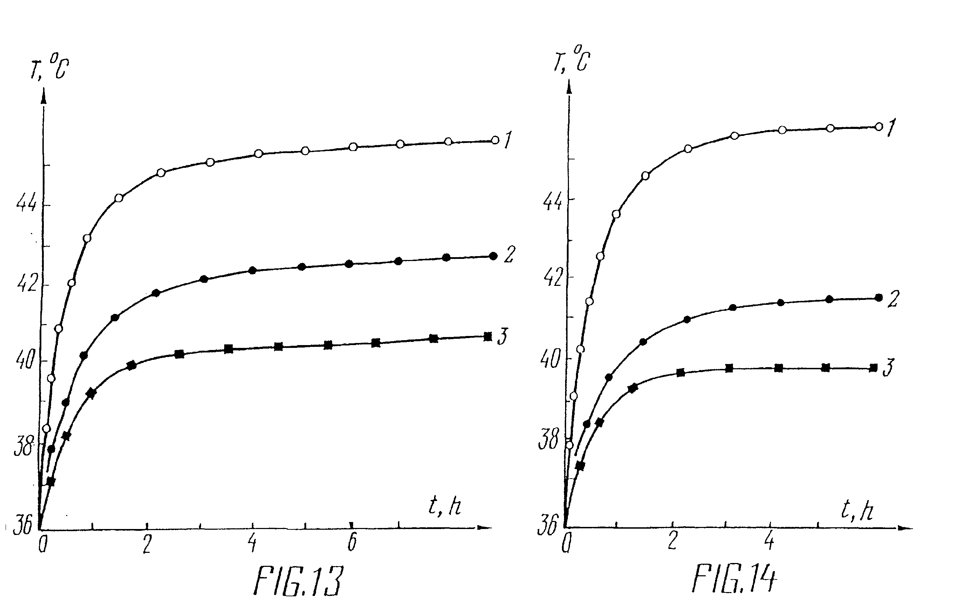

A study of the temperature profile during operation with this heating pad showed that heating takes place locally under the heating cell (Fig. 4). The difference between the temperature, which is under the heating cell, and that between the heating cells reaches 5°C.

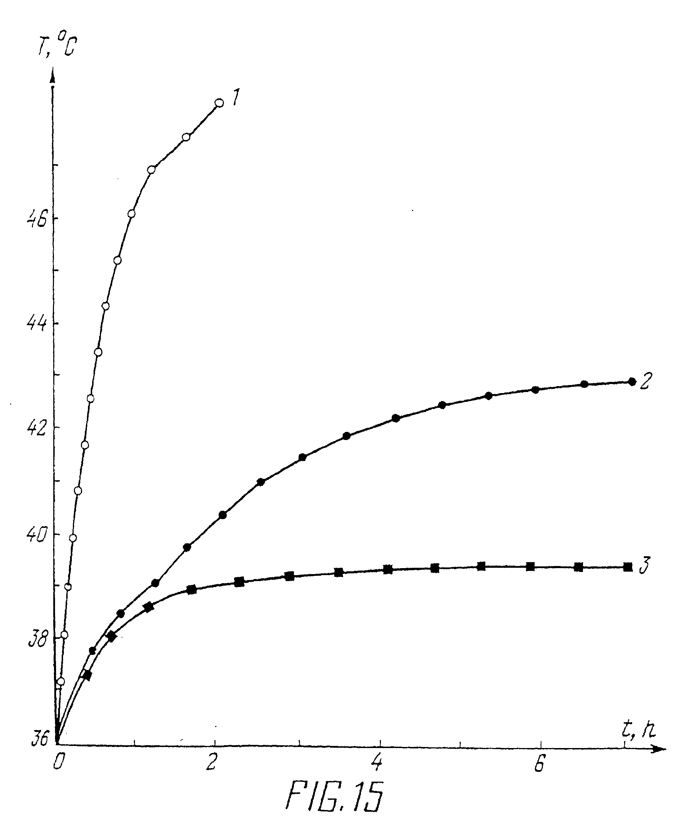

When a heat-distributing layer is used, there is a substantial smoothing of the temperature profile (Fig. 5), which is characterized by a reduction of the temperature under the heating cell and an increase of the temperature between the heating cells. Such a characteristic as dimensionless relative lateral heat conductivity is used as a characteristic of the heat-distributing properties. This characteristic is the ratio of the temperature difference under the heat-distributing layer between points under the heater and at a distance of 1 cm from the edge of the heating cell to the temperature difference between the temperature of the heater and at a point under the heat-distributing layer under the heating cell.

Thermocouples (10) are positioned at these points, see Fig. 3.

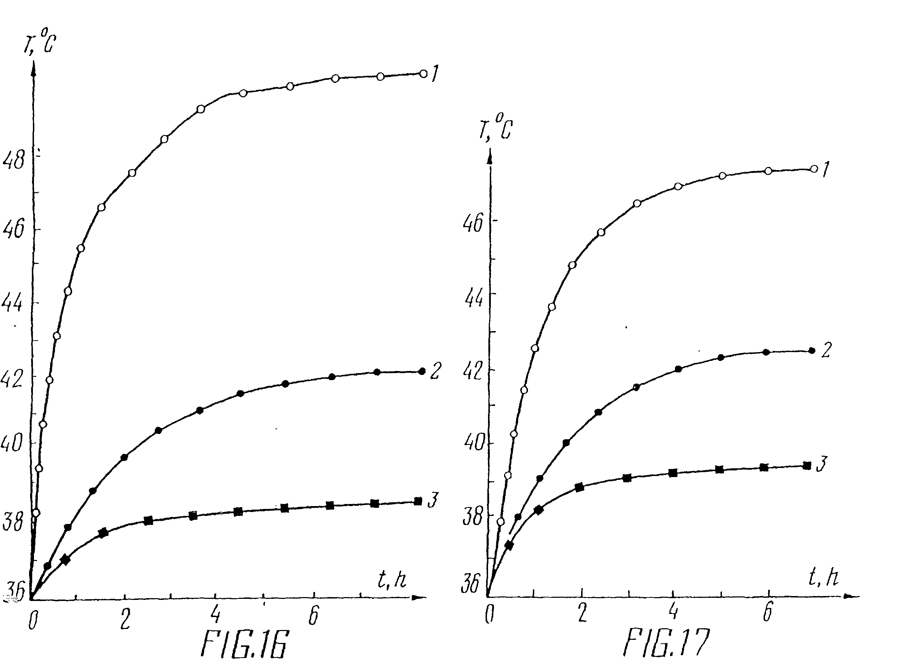

Fig. 6 shows data for a heat-distributing layer with a relative lateral heat conductivity of 2.4/0.1 of aluminum foil having a thickness of 0.04 mm.

Fig. 7 shows data for a heat-distributing layer with a relative lateral heat conductivity of 2/0.3 of brass grid made of 0.2 mm wire.

Fig. 8 shows data for a heat-distributing layer with a relative lateral heat conductivity within the range of 3.7/2.0 of Viskun fiber.

Fig. 9 shows data for a heat-distributing layer with relative lateral heat conductivity within the range of 4.5/0.9 of woven TU (carbon textile) fiber.

Fig. 10 shows data for a heat-distributing layer with a relative lateral heat conductivity within the range of 3.6/1.6 of woven RVTU material.

Fig. 11 shows data for a heat-distributing layer with a relative lateral heat conductivity of about 5.4/1.8 of woven polyester material in which finely dispersed particles of nickel with a specific resistance of 1.5 ohms are included in oriented fibers.

Fig. 12 shows data for a heat-distributing layer with a relative lateral heat conductivity of about 5.3/1.2 of woven arimide material in which finely dispersed particles of nickel with a specific resistance of 0.8 ohm are included in oriented fibers.

Fig. 13 shows data for a heat-distributing layer with a relative lateral heat conductivity of about 4.6/2.8 of Phenylon textile material in which finely dispersed particles of nickel with a specific resistance of 0.7 ohm are included in oriented fibers.

Fig. 14 shows data for a heat-distributing layer with a relative lateral heat conductivity of about 3.8/4.2 of woven polymer material in which finely dispersed particles of aluminum (50% by weight) are included in oriented fibers.

Fig. 15 shows data for a heat-distributing layer with a relative lateral heat conductivity of about 3.3/9.1 of nonwoven polymer material in which finely dispersed (5-20 µm) particles of BAU carbon are included.

Fig. 16 shows data for a heat-distributing layer with a relative lateral heat conductivity of about 2.5/8.0 of nonwoven polymer material in which finely dispersed (5-20 µm) particles of AG-3 carbon are included.

Fig. 17 shows data for a heat-distributing layer with a relative lateral heat conductivity of about 3.4/4.7-9.1 of nonwoven polymer material in which finely dispersed particles of SKT-125 carbon (surface density 125 g/m²) are included.

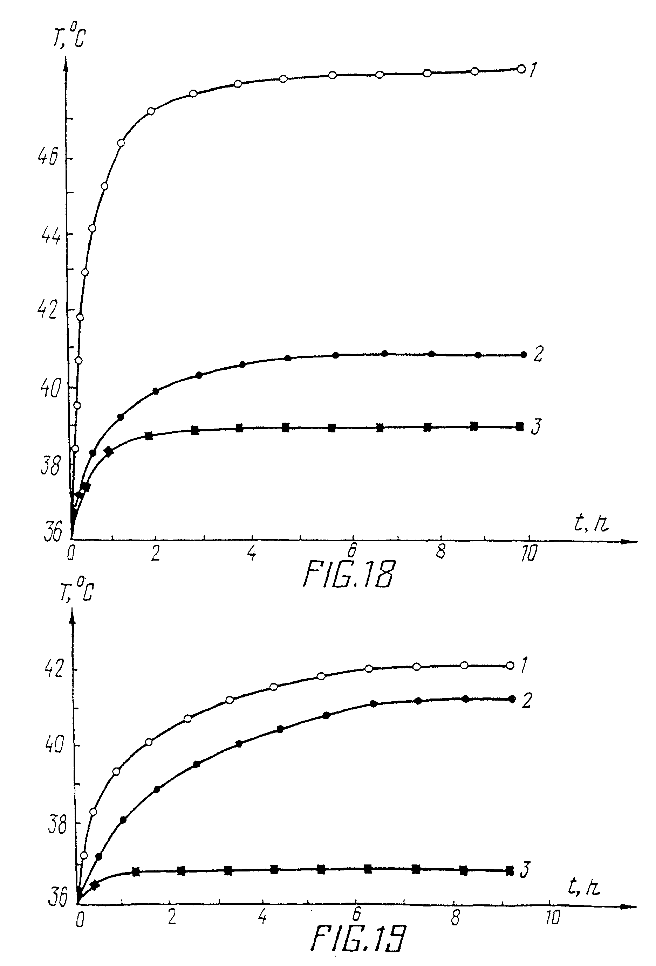

Fig. 18 shows data for a heat-distributing layer with a relative lateral heat conductivity of about 3.7/5.5 of nonwoven polymer material in which finely dispersed particles of SKT-250 carbon (surface density 250 g/m²) are included.

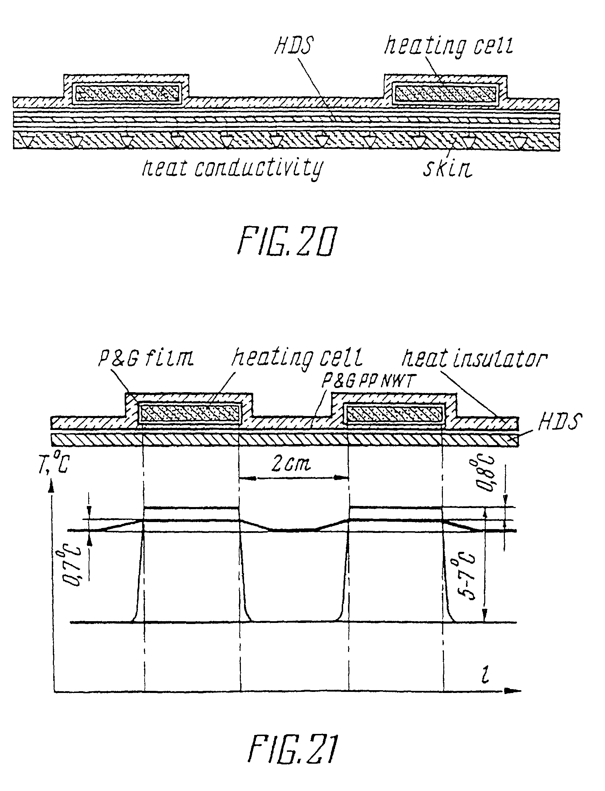

Fig. 19 shows data for a two-layer heat-distributing layer with a relative lateral heat conductivity of about 0.7/0.7, wherein aluminum foil which is 0.04 mm thick is used as the layer that is a good heat conductor, and TU textile material is used as the material that moderately conducts heat.

Fig. 20 shows a diagram of heat flows, and Fig. 21 shows a temperature profile for the case where, in addition to the two-layer heat-distributing layer described above, a heat-insulating layer is used as an outer insulating layer.

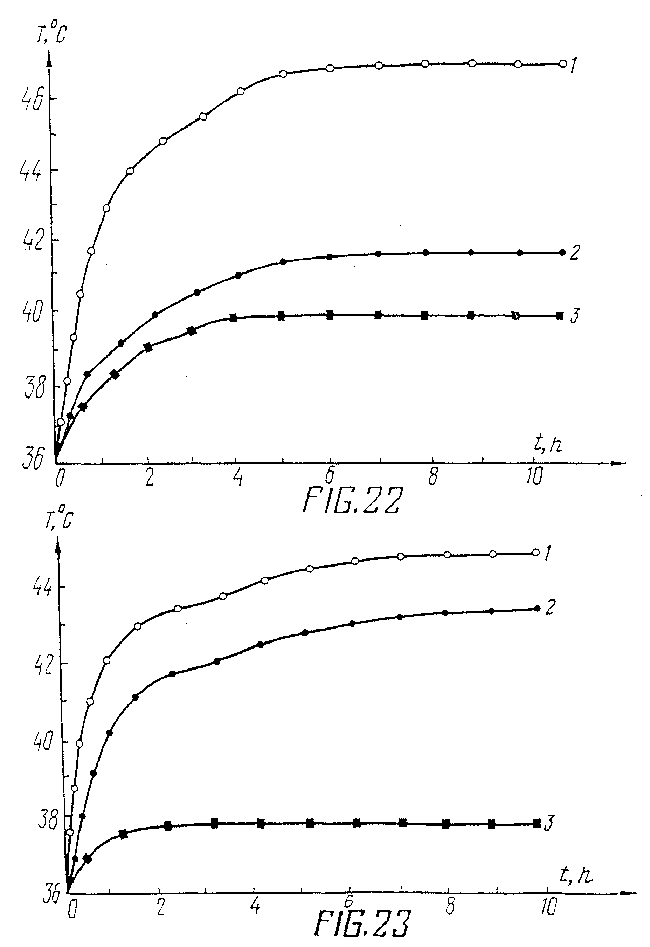

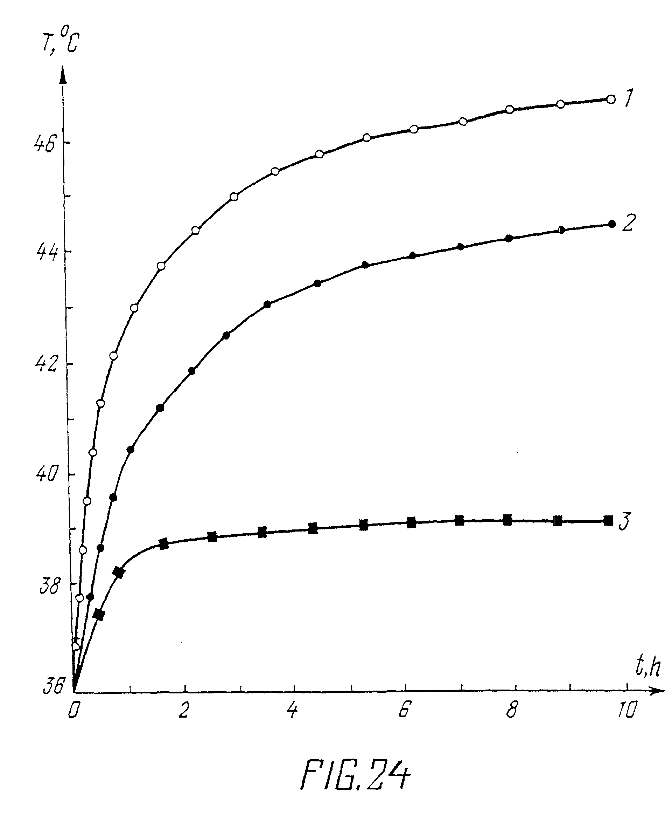

Fig. 22 shows data for the case where nonwoven material of polyacrylonitrile is used as the heat-insulating layer;

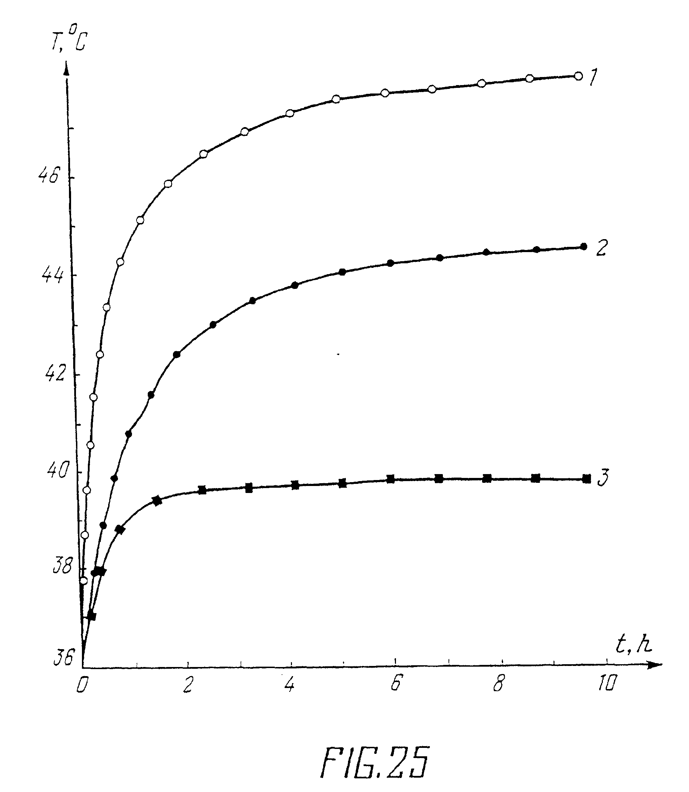

In Fig. 23 aluminum foil (0.015 mm) having a relative lateral heat conductivity of 1.3/1.1 is used as the material for the heat-conducting layer;

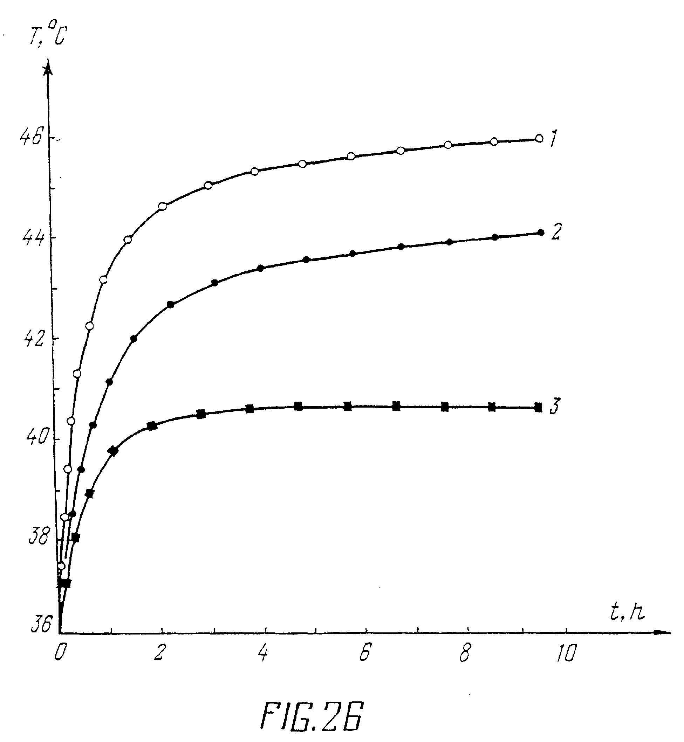

In Fig. 24 TU textile material having a relative lateral heat conductivity of 3.5/2.5 is used as the material for the heat-conducting layer;
In Fig. 25 Viskun textile material having a relative lateral heat conductivity of 3.5/3.1 is used as the material for the heat-conducting layer;
In Fig. 26 RVTU textile material having a relative lateral heat conductivity of 4.2/1.7 is used as the material for the heat-conducting layer;

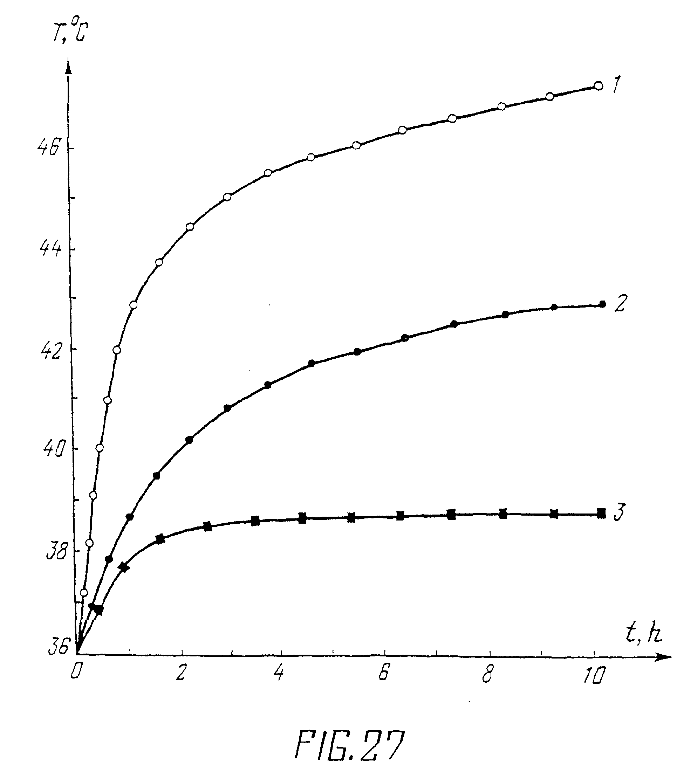

In Fig. 27 woven polyester material, with finely dispersed particles of nickel having a relative lateral heat conductivity of 3.5/5.1 included in oriented fibers, is used as the material for the heat-conducting layer;

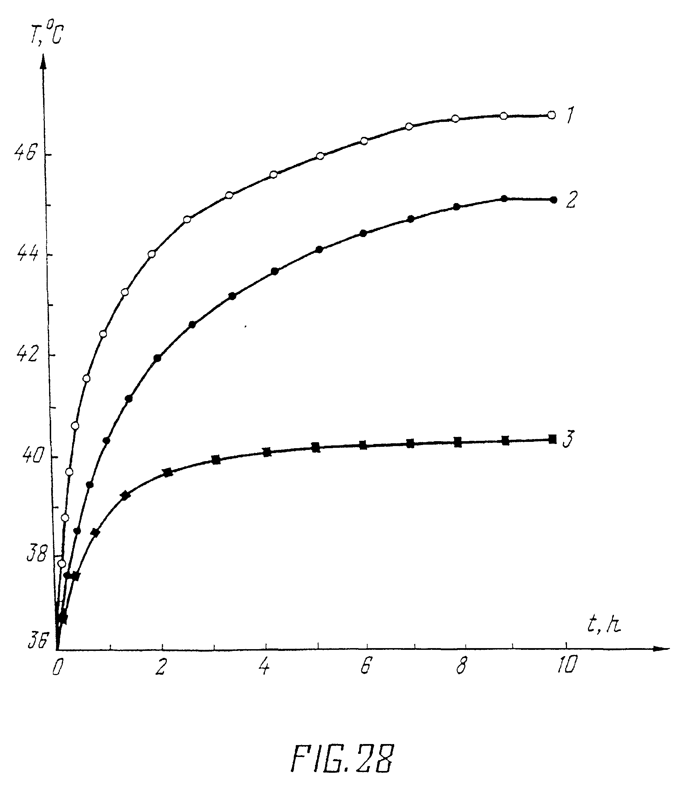

In Fig. 28 nonwoven material with a filling of finely dispersed boron nitride having a relative lateral heat conductivity of 3.9/1.6 is used as the material for the heat-conducting layer.

The obtained data are summarized in Table 1, presented below.

**Table 1**

| Material | Temperature of HC | Relative lateral heat conductivity |
|---|---|---|
| Aluminum foil of 0.04 thickness | 41.9 | 2.4/0.1 |
| Brass grid having a wire diameter of 0.2 mm | 42.2 | 2.0/0.3 |
| Viskun (C) | 46.7 | 3.7/2.0 |
| TU (C) | 47.0 | 4.5/0.9 |
| RVTU (C) | 43.9 | 3.6/1.6 |
| PE (Ni) with ρ = 1.5 ohms | 46.6 | 5.4/1.8 |
| arimide (Ni) with ρ= 0.8 ohm | 45.6 | 5.3/1.2 |
| Phenylon (Ni) with ρ= 0.7 ohm | 46.1 | 4.4/2.8 |
| PE (50% Al) | 46.5 | 3.8/4.2 |
| NM (BAU) | 49.5 | 3.3/9.1 |
| AG-3 | 49.7 | 2.5/8.0 |
| SKT-125 | 47.7 | 3.4/4.7 |
| SKT-250 | 48.3 | 3.7/5.5 |
| Al foil + TU | 41.4 | 0.7/0.7 |
| PAN+Al | 43.4 | 1.3/1.1 |
| PAN+Al+TU | 48.9 | 3.5/2.5 |
| PAN+Al+Viskun | 47.7 | 3.5/3.1 |
| PAN+Al+RVTU | 45.9 | 4.2/5.1 |
| PAN+textile with BN | 46.6 | 3.9/1.6 |
| PAN+Al+PE(Ni) | 51.0 | 3.5/1.6 |
| PAN (heat insulator) | 51.0 | 4.8/5.0 |
| NM - nonwoven material. PAN - nonwoven material polyacrylonitrile. | | |

## Claims

1. An individual heating device with a source of heat on the base of a heat-generating material, **characterized in that** it comprises a flexible heat-distributing layer which is positioned on one side of the source of heat and is in contact with the object being heated.

2. The heating device according to claim 1, **characterized in that** aluminum foil having a thickness of 0.04 mm is used as the heat-distributing layer.

3. The heating device according to claim 2, **characterized in that** the aluminum foil is perforated.

4. The heating device according to claim 1, **characterized in that** a brass grid of 0.2 mm wire is used as the heat-distributing layer.

5. The heating device according to claim 1, **characterized in that** a woven polymer material is used as the heat-distributing layer, the composition of said woven polymer material including finely dispersed particles of carbon.

6. The heating device according to claim 5, **characterized in that** Viskun fiber is used as the woven polymer material.

7. The heating device according to claim 5, **characterized in that** woven TU textile material is used as the woven polymer material.

8. The heating device according to claim 5, **characterized in that** the woven material RVTU is used as the woven polymer material.

9. The heating device according to claim 1, **characterized in that** a woven polymer material is used as the heat-distributing layer, the composition of said woven polymer material including finely dispersed particles of metal.

10. The heating device according to claim 9, **characterized in that** a woven polyester material is used as the woven polymer material, and the metal is nickel.

11. The heating device according to claim 9, **characterized in that** woven material Phenylon is used as the woven polymer material, and the metal is nickel.

12. The heating device according to claim 9, **characterized in that** the metal is aluminum.

13. The heating device according to claim 1, **characterized in that** a nonwoven polymer material is used as the heat-distributing layer, the composition of the nonwoven polymer material including finely dispersed (5-20 µm) particles of carbon (50% by weight).

14. The heating device according to claim 13, **characterized in that** the particles of carbon are particles of BAU carbon.

15. The heating device according to claim 13, **characterized in that** the particles of carbon are particles of AG-3 carbon.

16. The heating device according to claim 13, **characterized in that** the particles of carbon are particles of SKT-125 carbon having a surface density of 125 g/m².

17. The heating device according to claim 13, **characterized in that** the particles of carbon are particles of SKT-250 carbon having a surface density of 250 g/m².

18. The heating device according to claim 1, **characterized in that** the heat-distributing layer is two-layer, wherein the layer in contact with the source of heat is heat-conductive, and the layer in contact with the object being heated is moderately heat-conductive.

19. The heating device according to claim 18, **characterized in that** the high heat-conducting layer is aluminum foil having a thickness of 0.04 mm, while the moderate heat-conducting material is woven TU textile material having a relative lateral heat conductivity of about 0.7/0.7.

20. The heating device according to claim 18, **characterized in that** it comprises an upper heat-insulating layer of polyacrylonitrile nonwoven material and the source of heat is a phase change material.

21. The heating device according to claim 20, **characterized in that** the heat-conducting layer is aluminum foil (0.015 mm) having a relative lateral heat conductivity of 1.3/1.1.

22. The heating device according to claim 20, **characterized in that** the heat-conducting layer is woven TU textile material having a relative lateral heat conductance of 3.5/2.5.

23. The heating device according to claim 20, **characterized in that** the heat-conducting layer is woven Viskun material having a relative lateral heat conductivity of 3.5/3.1.

24. The heating device according to claim 20, **characterized in that** the heat-conducting layer is woven RVTU material having a relative lateral heat conductivity of 4.2/1.7.

25. The heating device according to claim 20, **characterized in that** the heat-conducting layer is woven polyester material, wherein finely dispersed particles of nickel having a relative lateral heat conductivity of 3.5/5.1 are included in oriented fibers.

26. The heating device according to claim 20, **characterized in that** the heat-conducting layer is nonwoven material with a filling of finely dispersed boron nitride having a relative lateral heat conductivity of 3.9/1.6.
